(19) Europäisches Patentamt / European Patent Office / Office européen des brevets

(11) **EP 4 256 325 B1**

(12) **EUROPEAN PATENT SPECIFICATION**

(45) Date of publication and mention
of the grant of the patent:
**07.05.2025 Bulletin 2025/19**

(21) Application number: **21831411.0**

(22) Date of filing: **01.12.2021**

(51) International Patent Classification (IPC):
**G01N 33/24** (2006.01)  **E21B 43/20** (2006.01)
**H01J 49/26** (2006.01)

(52) Cooperative Patent Classification (CPC):
**G01N 33/241; E21B 43/20;** G01N 2223/616;
H01J 49/26

(86) International application number:
**PCT/US2021/061405**

(87) International publication number:
**WO 2022/119920 (09.06.2022 Gazette 2022/23)**

(54) **METHODS FOR LOW SALINITY ENHANCED OIL RECOVERY**

VERFAHREN FÜR ERHÖHTE ÖLGEWINNUNG MIT NIEDRIGEM SALZGEHALT

PROCÉDÉS DE RÉCUPÉRATION DE PÉTROLE AMÉLIORÉE, À FAIBLE SALINITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.12.2020 US 202063120187 P**

(43) Date of publication of application:
**11.10.2023 Bulletin 2023/41**

(73) Proprietor: **BP Corporation North America Inc.**
**Houston, TX 77079 (US)**

(72) Inventors:
• **ZENG, Huang**
**Houston, TX 77079 (US)**
• **WICKING, Christianne, Clare**
**Houston, TX 77079 (US)**
• **TESSAROLO, Nathalia**
**Houston, TX 77079 (US)**
• **COUVES, John, William**
**Houston, TX 77079 (US)**
• **COLLINS, Ian, Ralph**
**Houston, TX 77079 (US)**

(74) Representative: **Boult Wade Tennant LLP**
**Salisbury Square House**
**8 Salisbury Square**
**London EC4Y 8AP (GB)**

(56) References cited:
US-A- 5 741 707

• AKSULU HAKAN ET AL: "Evaluation of Low-Salinity Enhanced Oil Recovery Effects in Sandstone: Effects of the Temperature and pH Gradient", ENERGY & FUELS, vol. 26, no. 6, 21 June 2012 (2012-06-21), WASHINGTON, DC, US., pages 3497 - 3503, XP055895583, ISSN: 0887-0624, DOI: 10.1021/ef300162n
• CHAVAN MUKUL ET AL: "Low-salinity-based enhanced oil recovery literature review and associated screening criteria", PETROLEUM SCIENCE, vol. 16, no. 6, 28 May 2019 (2019-05-28), Heidelberg, pages 1344 - 1360, XP055895638, ISSN: 1672-5107, DOI: 10.1007/s12182-019-0325-7
• "Data report: refined method for calculating percentages of kaolinite and chlorite from X-ray diffraction data, with application to the Nankai margin of southwest Japan", 4 November 2011, INTEGRATED OCEAN DRILLING PROGRAM, article GUO J. ET AL: "Data report: refined method for calculating percentages of kaolinite and chlorite from X-ray diffraction data, with application to the Nankai margin of southwest Japan", XP055895637, DOI: 10.2204/iodp.proc.314315316.201.2011

EP 4 256 325 B1

EP 4 256 325 B1

**Description**

**TECHNICAL FIELD**

The present disclosure relates generally to systems and methods for enhanced oil recovery (EOR). More particularly, the present disclosure relates to compositions and methods for assessing a reservoir rock response to low salinity water.

**BACKGROUND**

Waterflooding is one of the most common and widely used oil recovery methods practiced in the oil industry since the 1930s. Conventionally, waterflooding has been considered as a physical oil recovery process that serves two primary functions: (1) to maintain reservoir pressure, and (2) to displace oil from the reservoir pore space toward the producing well by viscous forces.

Low salinity waterflooding is an EOR method in which the salinity of the injected brine during a waterflood is reduced to below about 10,000 ppm total dissolved solids (TDS). The salinity reduction causes production of additional oil. It is thought that the EOR from low salinity waterflooding is dependent on a complex relationship between the salinity, composition of the crude oil in place, and the minerals present in the reservoir. However, the mechanism is widely debated in the open literature and not fully agreed upon. It is generally accepted that low salinity waterflooding leads to a wettability alteration of the rock surface, but the underlying microscopic mechanism is still not entirely understood. An ongoing need exists for novel methods and compositions for assessing the effectiveness of an enhanced oil recovery operation. Aksulu Hakan et al, Energy & Fuels 2012, vol. 26, no. 6, 3497-3503 relates to low-salinity oil recovery effects in sandstone. Chavan Mukul et al, Petroleum Science 2019, vol. 16, no. 6, 1344-1360 relates to low-salinity-based enhanced oil recovery. US 5 741 707 A relates to a method for quantitative analysis of earth samples. Guo J. et al, Integrated Ocean Drilling Program 2011 relates to a refined method for calculating percentages of kaolinite and chlorite from X-ray diffraction data.

**SUMMARY**

Disclosed herein is a method of assessing the response of a reservoir rock to low salinity water according to claim 1.

Also disclosed herein is a method of enhanced oil recovery from a resource-containing reservoir zone according to claim 11.

Embodiments described herein comprise a combination of features and characteristics intended to address various shortcomings associated with certain prior devices, systems, and methods. The foregoing has outlined rather broadly the features and technical characteristics of the disclosed embodiments in order that the detailed description that follows may be better understood. The various characteristics and features described above, as well as others, will be readily apparent to those skilled in the art upon reading the following detailed description, and by referring to the accompanying drawings. It should be appreciated that the conception and the specific embodiments disclosed may be readily utilized as a basis for modifying or designing other structures for carrying out the same purposes as the disclosed embodiments. It should also be realized that such equivalent constructions do not depart from the spirit and scope of the principles disclosed herein.

**BRIEF DESCRIPTION OF THE DRAWINGS**

For a detailed description of various exemplary embodiments, reference will now be made to the accompanying drawings, wherein like reference numerals represent like parts:

Figure 1 is a process flow diagram illustrating an embodiment of a method for obtaining samples for testing;

Figure 2A is a schematic depiction of a sample test for a low salinity response;

Figures 2B-2E are compilations of mass spectra for various rock samples treated as disclosed herein; and

Figure 3 is a plot of original oil in place as a function of the amount of catecholamine type structures.

2

## DETAILED DESCRIPTION

The following discussion is directed to various exemplary aspects. However, one skilled in the art will understand that the examples disclosed herein have broad application, and that the discussion of any aspect is meant only to be exemplary of that aspect, and not intended to suggest that the scope of the disclosure, including the claims, is limited to that aspect.

Certain terms are used throughout the following description and claims to refer to particular features or components. As one skilled in the art will appreciate, different persons may refer to the same feature or component by different names. This document does not intend to distinguish between components or features that differ in name but not function. The drawing figures are not necessarily to scale. Certain features and components herein may be shown exaggerated in scale or in somewhat schematic form and some details of conventional elements may not be shown in interest of clarity and conciseness.

In the following discussion and in the claims, the terms "including" and "comprising" are used in an open-ended fashion, and thus should be interpreted to mean "including, but not limited to...."

Disclosed herein are methods and compositions for use in EOR. In one or more aspects, the presently disclosed methods and compositions are for use in assessing the potential of a reservoir to yield additional natural resources (e.g., hydrocarbons) by flooding the reservoir with a low salinity fluid. The methods and compositions disclosed herein may provide one or more indicators that a reservoir, which previously produced a natural resource, such as a hydrocarbon, may provide an additional amount of that natural resource when treated with a low salinity fluid. In such aspects, the additional resources obtained from treatment with a low salinity fluid may range from about 1% to about 20% of the original amount of resources obtained from the reservoir or the original amount of oil in place (OOIP). Alternatively, the additional resources may be recovered in an amount of greater than about 1%, alternatively greater than about 5%, alternatively greater than about 10% or alternatively greater than about 15% of the OOIP based on total weight of OOIP. It is to be understood, the methods disclosed herein are utilized in wellbores or formations that previously had resources removed utilizing other methods (e.g., high salinity water flooding). Herein, the methods and compositions disclosed assess the Low Salinity Response (LSR) of the wellbore or formation.

Herein, the term "low salinity fluid" refers to a fluid (e.g., aqueous fluid) having a total dissolved solids (TDS) content of less than about 20,000 parts per million volume (ppmv), alternatively less than about 15,000 ppmv, or alternatively less than about 10,000 ppmv. Herein, the TDS refers to a measure of the dissolved combined content of all inorganic and organic substances present in a liquid in molecular, ionized, or micro-granular suspended form.

In one or more aspects, the low salinity fluid is a low salinity water having a TDS content in the range of from about 200 ppmv to about 10,000 ppmv, alternatively from about 500 ppmv to about 10,000 ppmv, alternatively from about 500 ppmv to about 7,500 ppmv, or alternatively from about 500 ppmv to about 5,000 ppmv.

The low salinity aqueous fluid may be a fresh water or a brackish water. Fresh water may be obtained from a river, lake, or an aquifer, and have a TDS content of less than about 1,500 ppm. Brackish water may be obtained from an estuarine river source, an inland sea, or an aquifer. In addition, certain produced waters may be of sufficiently low salinity that they may be employed as the low salinity water. Alternatively, the low salinity water may be a desalinated water produced by a desalination plant, such as a desalination plant employing a membrane desalination process such as reverse osmosis or forward osmosis (also referred to as direct osmosis) process. Waters that may be used as feed to a membrane desalination plant include seawater, brackish waters (for example, estuarine, aquifer or produced waters), saline aquifer waters, saline-produced waters or a combination thereof. The use of produced water as a feed to the desalination plant is advantageous in locations where there are restrictions on disposal of produced water. Seawater may also be a suitable feed water for a desalination plant. In such aspects, suitable seawater may be characterized as being inland seas having from about 15,000 ppmv to about 40,000 ppmv TDS, or oceanic seas, having from about 30,000 ppmv to 45,000 ppmv TDS.

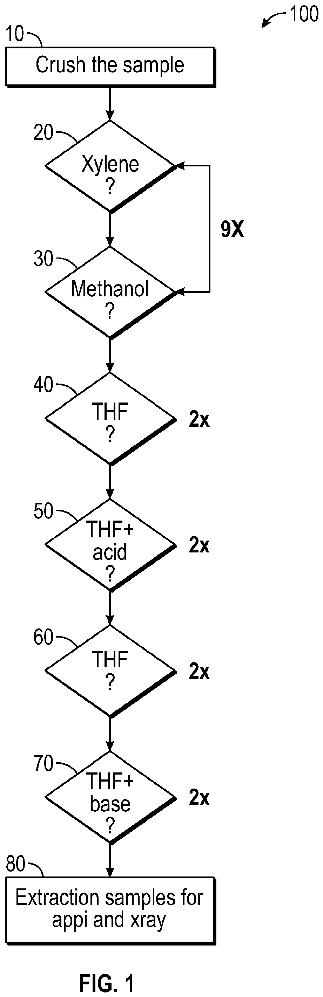

An aspect of a method of the present disclosure is depicted in Figure 1. In an aspect, a method for assessing the LSR of a reservoir, 100, comprises obtaining a sample of the reservoir or formation of interest. Such reservoir samples may be termed formation core samples or reservoir plugs.

Formation core samples may be acquired using any suitable methodology. For example, a formation core sample may be obtained by actuating a formation coring tool in a subterranean wellbore. The formation core sample may be retrieved to the surface. The formation core sample acquired may include any suitable formation core sample. For example, the formation core sample may include a fresh state formation core sample. By "fresh state" it is meant that the formation core sample is in an "as received" condition not having undergone sample preparation procedures such as cleaning, oil or brine flushing or re-saturation, and/or aging with various reservoir fluids. In an aspect, the formation core sample may comprise any number of minerals including but not limited to quartz, kaolinite, illite, mica, smecite, chlorite, feldspar, plagioclase, calcite, or a combination thereof.

In an aspect, the method further comprises crushing the formation core sample, 10, using any suitable methodology to form a crushed formation core sample. The method may further comprise washing the crushed formation core sample with a first series of solvents to form a first series of solvent extracts. In an aspect, the first series of solvents comprise a nonpolar solvent having a relative polarity of less than about 2.0, alternatively from about 0.0 to about 2.5 or alternatively from about 0.0 to about 2.0. In an aspect, the crushed formation core sample is washed with a nonpolar solvent selected from the group consisting of cyclohexane, pentane, heptane, hexane, carbon tetrachloride, carbon disulfide, p-xylene, toluene, benzene, ether, methyl-t-butyl ether, diethylamine, dioxane, and a combination thereof. In one or more aspects, the crushed formation core sample is washed with xylene, 20. The crushed formation core sample may be washed a plurality of times, denoted n, to generate n xylene wash samples where n ranges from 2 to 20, alternatively from 2 to 15 or alternatively from 2 to 10. In one or more aspects, n is 9. The xylene wash samples may each be each stored separately in a suitable container. In an aspect, the crushed formation core sample having been subjected to washing with a nonpolar solvent is termed a "washed, crushed" formation core sample. For each aspect disclosed herein, washing of a material, extraction of a material or contacting of a material (e.g., core formation sample) with a solution (e.g., solvent, treating fluid) may be carried out for any suitable time period, for example for from about 1 minute to about 60 minutes, alternatively from about 1 minute to about 20 minutes or alternatively from about 1 minute to about 5 minutes.

The activity disclosed (e.g., washing, extraction) may be carried out under ambient conditions (e.g., room temperature and pressure) in any suitable vessel or container. Means of agitating the materials that are being contacted, washed, treated such as stirring or shaking may be employed as is suitable. As would be understood by one of ordinary skill in the art, contacting of a formation core sample (solid) with any solution (e.g., solvent) results in a solid-liquid mixture that may be separated using any suitable methodology (e.g., gravity separation, centrifugation, filtration).

In an aspect, the method further comprises washing the washed, crushed formation core sample with a polar solvent. 30. In an aspect, the polar solvent has a relative polarity of greater than about 3.5, alternatively from about 3.6 to about 8.0, or alternatively from about 4.0 to about 8.0. In an aspect, the washed crushed formation core sample is washed with a polar solvent such as a $C_1$ to $C_{10}$ alcohol, or alternatively, a $C_1$ to $C_5$ alcohol. In some aspects, the alcohol can be methanol, ethanol, propanol, butanol, n-propanol, iso-propanol, n-butanol, tert-butanol, or a combination thereof. In an aspect, the polar solvent is selected from the group consisting of dimethyl sulfoxide, acetonitrile, 3-pentanol, 2-pentanol, butanol, cyclohexanol, 1-octanol, 2-propanol, 1-heptanol, acetone, diethylene glycol, methanol, ethanol, propanol, butanol, n-propanol, iso-propanol, n-butanol, tert-butanol, and a combination thereof. In one or more aspects, the washed crushed formation core sample is washed with methanol. Washing of the washed, crushed formation core sample may be carried out a plurality of times, denoted n, to generate n methanol wash samples where n ranges from 2 to 20, alternatively from 2 to 15, or alternatively from 2 to 10. In one or more aspects, n is 9. The polar wash samples (e.g., wash methanol) may be each stored separately in a suitable container. In an aspect, the washed crushed formation core sample having been subjected to washing with the polar solvent, as described herein, is termed an "extracted" formation core sample.

In an aspect, the extracted formation core sample, is subject to treatment with a series of treating solutions. In an aspect, the extracted formation core sample is contacted with a first treating solution comprising a solvent having a relative polarity of from about 3.5 to about 4.5, alternatively from about 3.5 to about 4.2 or alternatively about 4.0, 40. In an aspect, the first treating solution comprises an aprotic solvent, alternatively an aliphatic cyclic ether. In an aspect, the first treating solution comprises dimethyl ether, diethyl ether, a dipropyl ether, a dibutyl ether, methyl ethyl ether, a methyl propyl ether, a methyl butyl ether, tetrahydrofuran, a substituted tetrahydrofuran, a dihydrofuran, a substituted dihydrofuran, 1,3-dioxolane, a substituted 1,3-dioxolane, tetrahydropyran, a substituted tetrahydropyran, a dihydropyran, a substituted dihydropyran, pyran, a substituted pyran, a dioxane, a substituted dioxane, or a combination thereof. In an aspect, the first treating solution comprises tetrahydrofuran. The extracted formation core sample may contacted with the first treating solution a plurality of times, denoted n, to generate n first treated samples where n is ranges from 1 to 10, alternatively from 1 to 5 or alternatively from 2 to 5. In one or more aspects, n is 2. Each contact of the first treating solution with the extracted formation core generates a first treated solution which may each be stored separately in a suitable container. The extracted formation core sample after contact with the first treating solution is termed a treated formation core sample.

In an aspect, the method of the present disclosure further comprises contacting the treated formation core sample with a second treating solution comprising a solvent and an acid, 50. In an aspect, the solvent in the second treating solution is the same as the solvent in the first treating solution. Alternatively, the solvent in the second treating solution has a relative polarity index in the range disclosed for the solvent in the first treating solution but otherwise differs from the solvent of the first treating solution. In an aspect, the second treating solution comprises tetrahydrofuran. In an aspect, the second treating solution also comprises an acid. In an aspect, the second treating solution comprises formic acid, acetic acid, propanic acid, butanoic acid, citric acid, or a combination thereof. Alternatively, the second treating solution comprises formic acid. The second treating solution may contain a ratio of solvent to acid ranging from about 0.5 weight percent (wt.%) to about 20 wt.%, alternatively from about 0.5 wt.% to about 10 wt.%, or alternatively from about 0.5 wt.% to 2 wt.% based on the total weight of the treating solution.

The treated formation core sample may be contacted with the second treating solution a plurality of times, denoted n, to generate n twice-treated samples where n ranges from 1 to 10, alternatively from 1 to 5 or alternatively from 2 to 5. In one or more aspects, n is 2. The treated formation core sample having been contacted with a second treating solution is termed a "twice-treated" formation core sample (2XFCS). Each contact of the second treating solution with the treated formation core generates a twice-treated solution which may each be stored separately in a suitable container.

In an aspect, a method of the present disclosure further comprises contacting the 2XFCS with the first treating solution, 60. The 2XFCS may be contacted with the first treating solution a plurality of times, denoted n, to generate n washed 2XFCS where n ranges from 1 to 10, alternatively from 1 to 5, or alternatively from 2 to 5. In one or more aspects, n is 2. The 2XFCS having been contacted with the first treating solution is termed a washed twice-treated formation core sample (W-2XFCS). Each contact of the first treating solution with the 2XFCS results in a washed twice-treated sample which may each be stored separately in a suitable container.

In an aspect, a method of the present disclosure further comprises contacting the W-2XFCS with a third treating solution comprising a solvent and a base, 70. In an aspect, the solvent in the third treating solution is the same as the solvent in the first treating solution. Alternatively, the solvent in the third treating solution has a relative polarity index in the range disclosed for the solvent in the first treating solution but otherwise differs from the solvent of the first treating solution. In an aspect, the third treating solution comprises tetrahydrofuran. In an aspect, the third treating solution further comprises a base. Nonlimiting examples of bases suitable for use in the third treating solution includes potassium hydroxide, sodium hydroxide, pyridine, alkanamines, imidazole, benzimidazole, histidine, guanidine, phosphazene, hydroxides of quaternary ammonium cations, or a combination thereof. Alternatively, the third treating solution comprises tetramethylammonium hydroxide (TMAH) as the base. The third treating solution may contain a ratio of solvent to base ranging from about 0.5 wt.% to about 20 wt.%, alternatively from about 0.5 wt.% to about 10 wt.%, or alternatively from about 0.5 wt.% to about 2 wt.%. Each contact of the third treating solution with the W-2XFCS generates an extraction solution, which may each be stored separately in a suitable container. The W-2XFCS may be contacted with the third treating solution a plurality of times, denoted n, to generate n extraction samples where n ranges from 1 to 10, alternatively from 1 to 5 or alternatively from 2 to 5. In one or more aspects, n is 2. The W-2XFCS once treated with the third treating solution results in a depleted formation core sample.

In an aspect, the method further comprises subjecting the extraction samples to analysis by mass spectrometry to determine the amount of catecholamine type structures (CTS) present in the extraction samples, 80. Herein, CTS refers to molecules having the general formula $C_xH_yN_mO_{x-3}$ where x is from 10 to 30, y is from 22 to about 62, and m is from 1 to 2. In an alternative aspect, the CTS refers to molecules having the general formula $C_xH_yO_{x-3}$ where x is from 10 to 30 and y is from 22 to 62. In a further aspect, the CTS has a double bond equivalent (DBE) ranging from 2 to 10, alternatively from 2 to 8, or alternatively from 2 to 6. The DBE, also termed the Degree of Unsaturation (DoU) or the extent of Hydrogen insufficiency, refers to the level of unsaturation or the amount of pi electrons present in the molecule. The DBE can be calculated utilizing formula (1);

$$DBE= C -(H/2) + (N/2) + 1$$

Formula 1

where C is the number of carbon atoms, N is the number of nitrogen atoms, and H is the number of hydrogen atoms.

Without being limited by theory, CTS molecules having the general structure disclosed herein may function to compatibilize the interface of formation surfaces with hydrocarbons located within the formation thus stabilizing the resource-rock interaction and reducing the LSR. In an aspect, the relative abundance of CTS in the extraction samples may be determined by any suitable methodology. For example, the relative abundance of CTS may be determined by mass spectrometry, alternatively high resolution mass spectrometry, alternatively high resolution mass spectrometry with an atmospheric phase photoionization mode (APPI).

Mass spectrometry (MS) is an analytical chemistry technique that helps identify the amount and type of chemicals present in a sample. Mass spectrometry works by ionizing chemical compounds to generate charged molecules or molecule fragments and measuring their mass-to-charge ratios. A mass spectrum (plural spectra) is a plot of the ion signal as a function of the mass-to-charge ratio. From spectra, the mass of the molecular ion and fragments are used to determine the elemental composition or isotopic signature of a compound and this information is used to elucidate the chemical structures of molecules. In APPI the principle is to use photons to ionize gas-phase molecules. After vaporization, the analyte interacts with photons emitted by a discharge lamp. These photons induce a series of gas-phase reactions that lead to ionization of the sample molecules. Several APPI sources have been developed and are commercially available.

The direct ionization of an analyte is generally characterized by a weak efficiency. This can be partially explained by the solvents which can absorb photons producing photoexcitation without ionization. The result is a reduction in the number of photons available for the direct ionization of the target compound, thus reducing the ionization efficiency. Thus, two distinct APPI sources are contemplated for use in analysis of the extraction samples; direct APPI and dopant APPI.

In an aspect, the LSR is proportional to the relative abundance of extractable CTS in the reservoir as determined by the amount of CTS in the extraction samples. Generally, the higher the amount of CTS present in the extraction samples the greater the tendency for the formation to yield additional natural resources (e.g., hydrocarbons) when treated with a low salinity water (i.e., high potential for a LSR).

In one or more aspects, the present of extractable CTS in the reservoir is indicative of the strength of the interaction between the crude oil and the mineral surface in the reservoir rock. In oil field applications, the fluid phases may be water, oil, and/or gas while the solid phase may be the minerals that make up the reservoir rock. The crude oil is "anchored" on the mineral surface due to the interaction between the polar compounds in the crude oil and the mineral surface. CTS is a group of organic molecules with the following structural characteristics: (1) an aromatic "core" which consists of one or more aromatic rings; (2) the presence of four or more functional groups (e.g. $-NH_2$, $-OH$) on the aromatic "core", where each of these functional groups can form one or more hydrogen bonds with other polar compounds in the crude oil or the hydroxyl groups ($-OH$) on the mineral surface. As a result, CTS molecules can act as "anchoring points" to keep the crude oil on the mineral surface. On the other hand, CTS molecules have different interaction with different minerals. Kaolinite has a very strong interaction with CTS molecules while the interaction between other minerals and CTS is relatively weak. The capability of the injected low salinity brine to remove crude oil from the mineral surface is strongly dependent on the amount of the CTS on the mineral surface as well as the amount of kaolinite in the reservoir rock. If the amount of CTS on the mineral surface is low, then the oil "anchored" on the mineral surface is limited, and there is not much "anchored" oil to be removed from the mineral surface. As a result, introducing a low salinity brine to the reservoir will not result enhanced oil recovery from the rock. In other words, "low salinity brine EOR effect" is not observed in this rock or the rock would be expected to have a poor LSR. This can be attributed to a situation where the reservoir rock simultaneously has (1) a high abundance of CTS molecules on the rock surface, and (2) a high abundance of kaolinite mineral, the amount of "anchored oil" on the rock surface is high, but the "anchored oil" cannot be released by a low salinity brine (hence a poor LSR) because there are too many "anchoring points," which strongly keep the oil on the rock surface. In this case, the "low salinity brine EOR effect" cannot be observed. In another instance, the reservoir rock simultaneously has (1) a high abundance of CTS molecules on the rock surface, and (2) a low abundance of kaolinite mineral, the amount of "anchored oil" on the rock surface is high. In such instances, because the interaction between the CTS molecules and non-kaolinite minerals is weak, this weak interaction can be broken by the low salinity brine resulting in the weakly "anchored" oil being released by a low salinity brine. As a result, the "low salinity brine EOR effect" can be observed in this case or a good LSR.

The present disclosure provides for a "low salinity brine EOR response" of a reservoir rock being related to the relative abundance of extractable CTS present in the reservoir rock. The relative abundance of extractable CTS present in the reservoir rock may designated as "high" when a relative abundance of extractable CTS ranges in amount from about 25 mole percent (mol.%) to about 95 mol.%, alternatively from about 25 mol.% to about 90 mol.%, or alternatively from about 40 mol.% to about 90 mol.%, where the mole percentage is based on the total abundance of the ionizable species revealed by the Atmospheric Pressure Photoionization High Resolution Mass Spectrometry (APPI-HRMS). Alternatively, the relative abundance of extractable CTS may be designated as low when a relative abundance of extractable CTS is less than about 25 mol.% where the mole percentage is based on the total abundance of the ionizable species revealed by the Atmospheric Pressure Photoionization High Resolution Mass Spectrometry (APPI-HRMS).

Reservoir rock may comprise a variety compounds including, but not limited, to quartz, kaolinite, illite, mica, smecite, chlorite, feldspar, plagioclase, calcite, or a combination thereof. In an aspect, the method further comprises determining the amount of kaolinite in the formation core sample.

Kaolinite refers to hydrous aluminum silicate clay with an extended sheet structure which can be regarded as having two constituents, a layer of tetrahedral $SiO_4$ and a layer of octahedral $OH^-$. The amount of kaolinite in the reservoir core formation sample may be determined using any suitable methodology. For example, the amount of kaolinite in the reservoir core formation sample may be determined by X-ray fluorescence analysis, X-ray diffraction analysis, scanning electron microscopy, or a combination thereof. In an aspect, the amount of kaolinite in the reservoir core formation sample is inversely proportional to the LSR such that as the amount of kaolinite in the reservoir core formation sample increases the LSR decreases. In an aspect, the reservoir core formation sample contains a low amount of kaolinite. Herein, a "low" amount refers to from about 0 weight percent (wt.%) to about 5 wt.%, alternatively less than about 5 wt.% or alternatively less than about 1 wt.% based on X-Ray Diffraction (XRD) analysis.

In an aspect, the methods of the present disclosure may be utilized in order to determine the potential for a reservoir to produce additional resources such as hydrocarbons. In such aspects, a formation core sample is obtained and treated as disclosed herein to obtain extraction samples and the percentage kaolinite in the formation. In an aspect, the formation core sample provides extraction samples having CTS in a relative abundance ranging from about 20 wt.% to about 90 wt.%, alternatively from about 40 wt.% to about 80 wt.% or alternatively from about 50 wt.% to about 80 wt.%. Additionally, the formation core may have an amount of kaolinite of less than about 2 wt.%, alternatively less than about 1 wt.%, or alternatively less than about 0.5 wt.%. In such aspects, the formation would be expected to exhibit a positive LSR wherein flooding of the formation with a low salinity water produces from about 0.1% to about 20% of the OOIP (e.g., hydrocarbons), alternatively from about 1% to about 15% of the OOIP, or alternatively from about 5% to about 15% of the OOIP.

In an aspect, a reservoir, being assessed as disclosed herein, is found to have a positive LSR. The methods of this disclosure may further comprise subjecting the hydrocarbon-bearing layer of the reservoir to a low salinity waterflood. In some aspects, the method comprises (and/or a control system is programmed to provide) injection of the low salinity injection fluid into the oil-bearing layer of the reservoir.

The low salinity injection water can be produced and the low salinity waterflood effected via any suitable methodology. In sandstone reservoirs, depending on the content and composition of clays in the formation rock, injection of a low salinity water may result in loss of injectivity. This loss of injectivity arises from formation damage in the near wellbore region of the injection well caused by swelling and migration of clays.

U.S. Patent No. 3208528 teaches the phenomenon of loss of permeability ("clay blocking") when clays or other mineral fines become dispersed by fresh water, and the dispersed particles bridge to partially dam interstices in the rock. In such aspects, treating a water-sensitive formation by varying the salinity of the injection water such that the salt content in the injected water is reduced gradually or in step-wise fashion while maintaining constant the ratio of total salt concentration to divalent cation salt concentration until the salt concentration in the water being injected has reached the desired degree of dilution can mitigate the risk of formation damage. Thus, this stepped or gradual reduction in salinity may be utilized to mitigate the risk of "salinity shock" and of permeability damage leading to a loss of injectivity. Accordingly, in some aspects, injection of low salinity water can be performed by gradually reducing a salinity of the injection water aqueous injection fluid to a desired salinity for a main phase.

In some aspects, the methods of the present disclosure result in more effective EOR due to the ability to assess the LSR of a formation and specifically determine if the formation has a CTS and kaolinite content that indicates a positive LSR.

**EXAMPLES**

The aspects having been generally described, the following examples are given as particular aspects of the disclosure and to demonstrate the practice and advantages thereof. It is understood that the examples are given by way of illustration and are not intended to limit the specification or the claims in any manner.

The methods disclosed herein were used to assess the LSR of different rock formations. Six formation samples were obtained. Sample A was a preserved reservoir rock which was produced from Reservoir I, and was filled with crude oil (Oil I). The rock matrix in Sample A is called "Rock 1". Sample B was another reservoir rock ("Rock 2") which has been solvent cleaned, then aged with Oil I at reservoir conditions; subsequently the rock was waterflooded with low salinity brine and then solvent cleaned. Sample C was an outcrop rock ("Rock 3") which has been solvent cleaned, then aged with Oil I at reservoir conditions; subsequently it was waterflooded with low salinity brine, and solvent cleaned. Sample D was the same outcrop rock ("Rock 3") which has been solvent cleaned, then aged with Oil A at reservoir conditions, then it was waterflooded with high salinity brine first; subsequently it was further waterflooded with low salinity brine, and solvent cleaned. Sample E was a preserved reservoir rock which was produced from Reservoir II, and was filled with crude oil (Oil II). The rock matric in Sample E is called "Rock 4". Sample F was another preserved reservoir rock which was produced from Reservoir II, and was waterflooded using low salinity brine and then solvent cleaned. The rock matrix in Sample F is also "Rock 4". Details of the procedure of rock aging, the Reservoir Condition waterflooding, as well as the solvent cleaning process can be found in previous publication: Webb, K., Lager, A. and Black C., Comparison of High/Low Salinity Water/Oil Relative Permeability, presented at the International Symposium of the Society of Core Analysts, Abu Dhabi, UAE, Oct. 29th to Nov. 2nd, 2008.

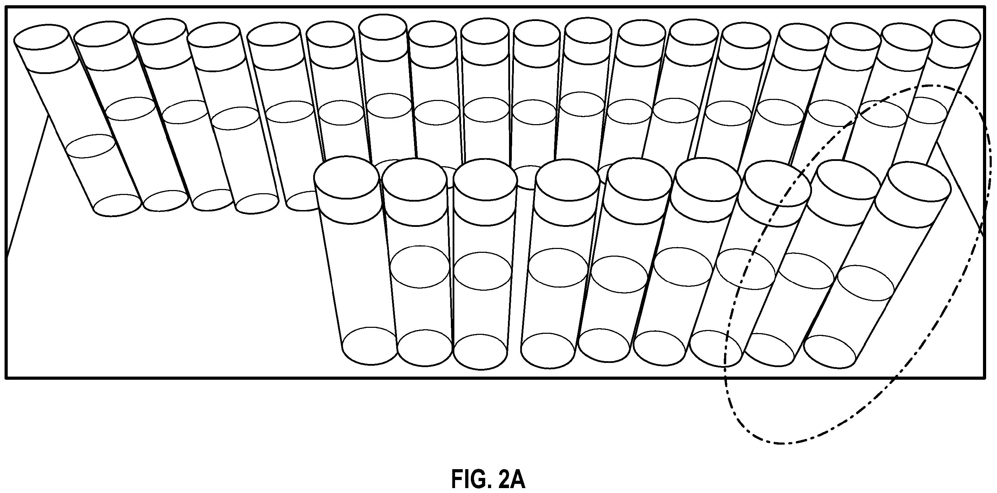

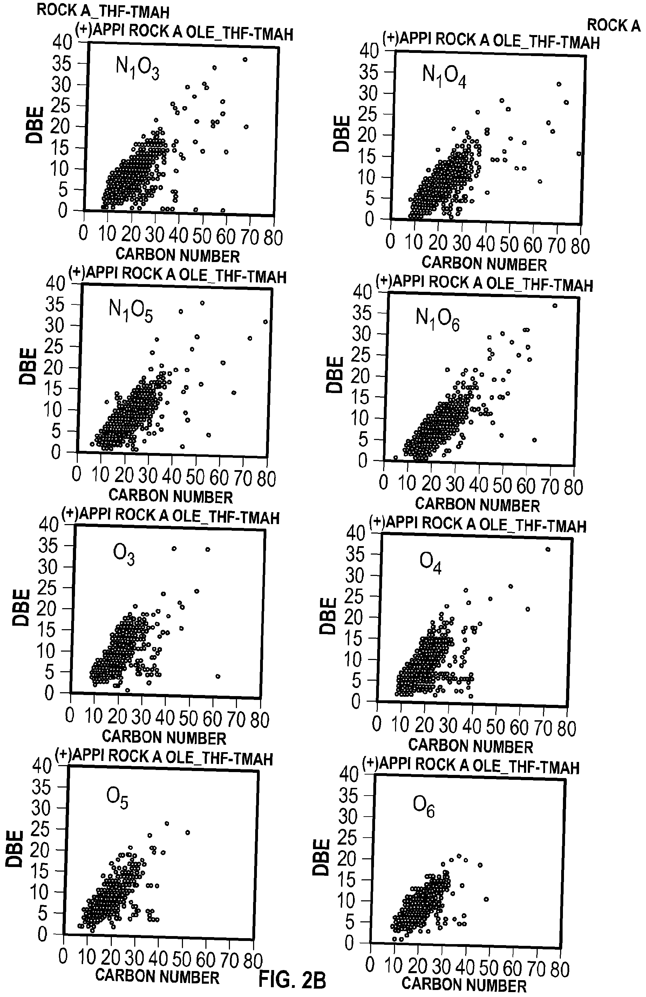

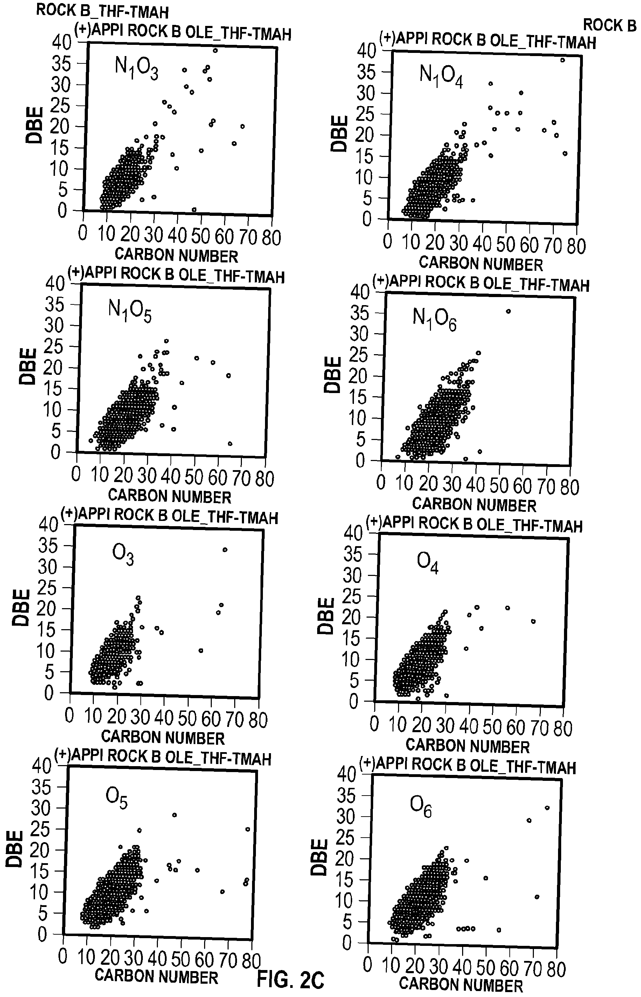

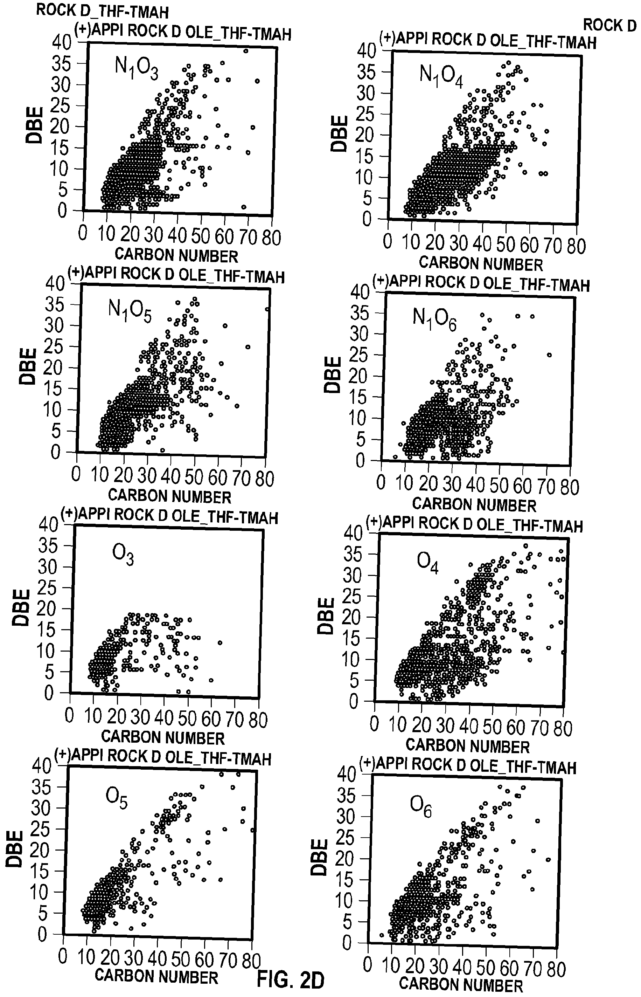

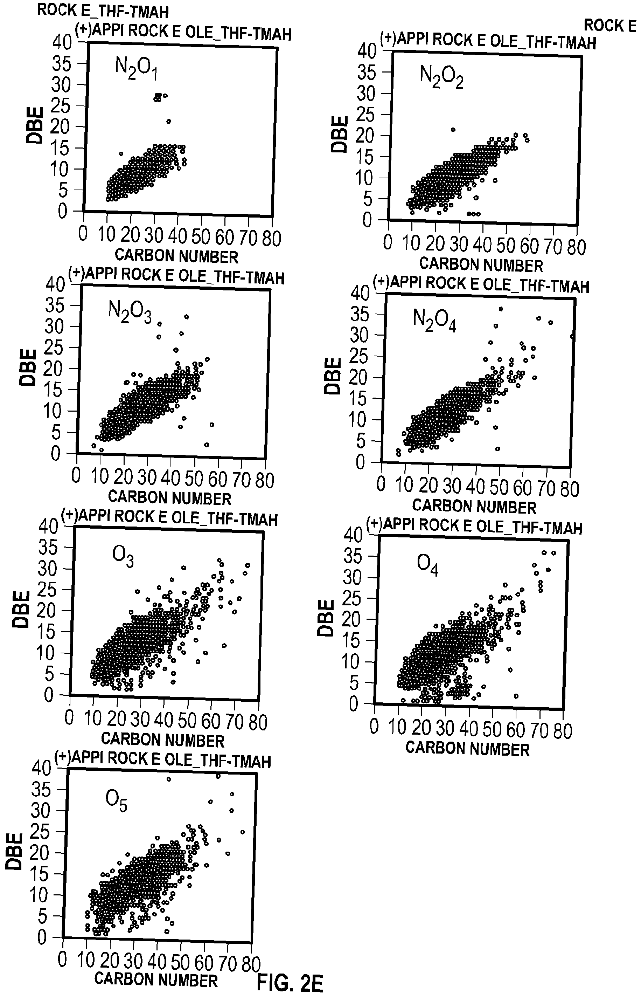

Samples A through F were crushed and then each sample was subjected to nine rounds of xylene extraction followed by methanol extractions. The samples were then extracted sequentially (i) twice with tetrahydrofuran (THF), (ii) twice with a THF-acid solution, (iii) twice with THF and finally (iv) twice with a THF-base solution. The samples following extraction with a THF-base solution were subjected to high resolution mass spectrometry-APPI. The mass spectra are presented in Figure 2.

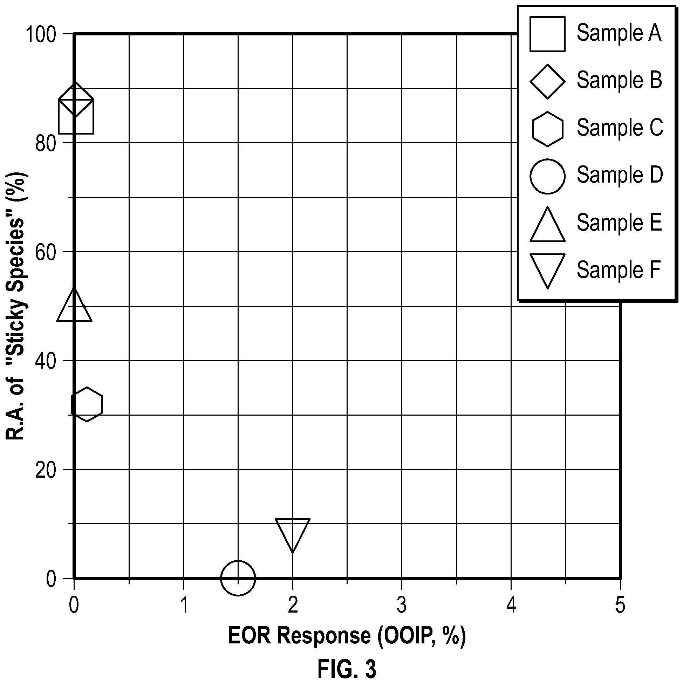

The EOR response in terms of OOIP were determined for Samples A-F are presented as a graph of relative abundance of CTS, Figure 3.

Rock 1, Rock 2, Rock 3 and Rock 4 were further analyzed by X-ray diffraction and the minerology of these samples are presented in Table 1.

TABLE 1

|  | Rock 1 | Rock 2 | Rock 3 | Rock 4 |
|---|---|---|---|---|
| Quartz | 86.1 | 89.1 | 95.7 | 95.6 |
| Kaolinite | 4.9 | 10.9 | 2.4 | 0.2 |
| Illite+Mica | 3.7 | 0.0 | 0.2 | 0.4 |
| Smectite | 0.0 | 0.0 | 0.0 | 1.1 |

(continued)

|  | Rock 1 | Rock 2 | Rock 3 | Rock 4 |
|---|---|---|---|---|
| Chlorite | 0.0 | 0.0 | 0.0 | 0.2 |
| K Feldspar | 3.6 | 0.0 | 1.1 | 2.6 |
| Plagioclase | 1.7 | 0.0 | 0.6 | 0.0 |
| Calcite | 0.0 | 0.0 | 0.0 | 0.0 |
| Clay_Wt. %_<2um | 2.1 | 1.5 | 2.3 | 2.1 |

The results demonstrate that the sample having the worst LSR, Sample A, also had the highest percentage kaolinite while the sample having the best LSR, Sample F, had the lowest percentage kaolinite.

While various aspects have been shown and described, modifications thereof can be made by one skilled in the art without departing from the spirit and teachings of the disclosure. The aspects described herein are exemplary only, and are not intended to be limiting. Many variations and modifications of the subject matter disclosed herein are possible and are within the scope of the disclosure. Where numerical ranges or limitations are expressly stated, such express ranges or limitations should be understood to include iterative ranges or limitations of like magnitude falling within the expressly stated ranges or limitations (e.g., from about 1 to about 10 includes, 2, 3, 4, etc.; greater than 0.10 includes 0.11, 0.12, 0.13, etc.). For example, whenever a numerical range with a lower limit, RL and an upper limit, RU is disclosed, any number falling within the range is specifically disclosed. In particular, the following numbers within the range are specifically disclosed: R=RL+k*(RU-RL), wherein k is a variable ranging from 1 percent to 100 percent with a 1 percent increment, i.e., k is 1 percent, 2 percent, 3 percent, 4 percent, 5 percent, ... 50 percent, 51 percent, 52 percent, ... , 95 percent, 96 percent, 97 percent, 98 percent, 99 percent, or 100 percent. Moreover, any numerical range defined by two R numbers as defined in the above is also specifically disclosed. Use of the term "optionally" with respect to any element of a claim is intended to mean that the subject element is required, or alternatively, is not required. Both alternatives are intended to be within the scope of the claim. Use of broader terms such as comprises, includes, having, etc. should be understood to provide support for narrower terms such as consisting of, consisting essentially of, comprised substantially of, etc.

Accordingly, the scope of protection is not limited by the description set out above but is only limited by the claims which follow, that scope including all equivalents of the subject matter of the claims. Each and every claim is incorporated into the specification as an aspect of the present disclosure. Thus, the claims are a further description and are an addition to the aspects of the present disclosure. The discussion of a reference is not an admission that it is prior art to the present disclosure, especially any reference that may have a publication date after the priority date of this application. The disclosures of all patents, patent applications, and publications cited herein are hereby incorporated by reference, to the extent that they provide exemplary, procedural, or other details supplementary to those set forth herein.

ADDITIONAL DESCRIPTION

The particular aspects disclosed above are illustrative only, as the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative aspects disclosed above may be altered or modified and all such variations are considered within the scope and spirit of the present disclosure. Alternative aspects that result from combining, integrating, and/or omitting features of the aspect(s) are also within the scope of the disclosure. While compositions and methods are described in broader terms of "having", "comprising," "containing," or "including" various components or steps, the compositions and methods can also "consist essentially of" or "consist of" the various components and steps. Use of the term "optionally" with respect to any element of a claim means that the element is required, or alternatively, the element is not required, both alternatives being within the scope of the claim.

Numbers and ranges disclosed above may vary by some amount. Whenever a numerical range with a lower limit and an upper limit is disclosed, any number and any included range falling within the range are specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an", as used in the claims, are defined herein to mean one or more than one of the element that it introduces. If there is any conflict in the usages of a word or term in this specification and one or more patent or other documents, the definitions that are consistent with this specification should be adopted.

Aspects disclosed herein include:

A first aspect which is a method of assessing the response of a reservoir rock to low salinity water according to claim 1.

A second aspect which is a method of enhanced oil recovery from a resource-containing reservoir zone according to claim 11.

Preferred aspects are indicated in the dependent claims.

## Claims

1. A method of assessing the response of a reservoir rock to low salinity water comprising:

   obtaining a formation core sample (10) of a reservoir rock from a reservoir (100);
   sequentially washing the formation core sample (10) with a first series of solvents comprising a nonpolar solvent followed by a polar solvent to form a first series of solvent extracts and an extracted formation core sample (10);
   sequentially washing the extracted formation core sample (10) with a treating solution to form a second series of solvent extracts and a cleaned formation core sample (10);
   generating a series of mass spectra of the second series of solvent extracts, wherein the relative abundance of the catecholamine-type structures (CTS) is determined using the series of mass spectra, wherein the CTS comprise organic molecules comprising an aromatic core consisting of one or more aromatic rings and four or more functional groups on the aromatic core, and wherein the CTS is selected from the group consisting of compounds having a general formula $C_xH_yN_mO_{x-3}$ where x is from 10 to 30, y is from 22 to 62, and m is from 1 to 2 and compounds having a general formula $C_xH_yO_{x-3}$ where x is from 10 to 30, and y is from 22 to 62;
   subjecting the formation core sample (10) to analysis by X-ray diffraction to generate a diffraction pattern, wherein the relative abundance of kaolinite is determined using the diffraction pattern; and
   assessing a response of the reservoir rock to low salinity water based on the percentage of kaolinite and the relative abundance of CTS, wherein the reservoir rock having a CTS of greater than 25 mol% produces an additional 0.01% to 20% amount of the original oil in place when contacted with low salinity water.

2. The method of claim 1, wherein the CTS has a double bond equivalent of from 2 to 10.

3. The method of claim 1, wherein the reservoir rock comprises quartz, kaolinite, illite, mica, smecite, chlorite, feldspar, plagioclase, calcite, or a combination thereof.

4. The method of claim 1, wherein the treating solution comprises a compound having a polarity index ranging from 0.175 to 0.300.

5. The method of claim 1, wherein the treating solution comprises an aprotic solvent or an aliphatic cyclic ether.

6. The method of claim 5, wherein the aliphatic cyclic ether comprises dimethyl ether, diethyl ether, a dipropyl ether, a dibutyl ether, methyl ethyl ether, a methyl propyl ether, a methyl butyl ether, tetrahydrofuran (40), a substituted tetrahydrofuran, a dihydrofuran, a substituted dihydrofuran, 1,3-dioxolane, a substituted 1,3-dioxolane, tetrahydropyran, a substituted tetrahydropyran, a dihydropyran, a substituted dihydropyran, pyran, a substituted pyran, a dioxane, a substituted dioxane, or a combination thereof.

7. The method of claim 1, wherein the treating solution comprises (i) tetrahydrofuran (40), and/or (ii) an acid or a base.

8. The method of claim 7, wherein the acid comprises, formic acid, acetic acid, propanic acid, butanoic acid, citric acid, or a combination thereof, or
   wherein the acid comprises formic acid.

9. The method of claim 7, wherein the base comprises potassium hydroxide, sodium hydroxide, pyridine, alkanamines, imidazole, benzimidazole, histidine, guanidine, phosphazene, hydroxides of quaternary ammonium cations, or a combination thereof, or wherein the base comprises tetramethylammonium hydroxide.

10. The method of claim 1, wherein the high resolution mass spectrometry comprises an atmospheric phase photoionization mode.

11. A method of enhanced oil recovery from a resource-containing reservoir (100) zone comprising:

obtaining a rock sample from the resource-containing reservoir (100) zone;

contacting the rock sample with solvents sequentially to form solvent extracts and a cleaned rock sample;

subjecting the cleaned rock sample to X-ray diffraction wherein X-ray diffraction identifies a percentage kaolinite of the cleaned rock sample;

subjecting the solvent extracts to mass spectrometry wherein the mass spectrometry identifies a percentage catecholamine-type structures (CTS) in at least one of the solvent extracts, wherein the CTS comprise organic molecules comprising an aromatic core consisting of one or more aromatic rings and four or more functional groups in the aromatic core, and wherein the CTS comprises compounds having a general formula $C_xH_yN_mO_{x-3}$ where x is from 10 to 30, y is from 22 to 62, and m is from 1 to 2 and compounds having a general formula $C_xH_yO_{x-3}$ where x is from 10 to 30, and y is from 22 to 62;

determining the reservoir rocks with high possibility of carrying positive response to low salinity water enhanced oil recovery method based on the percentage of kaolinite and the percentage of CTS, wherein a high possibility of positive response is **characterized by** having a CTS of greater than 25 mol%; and

initiating an enhanced oil recovery operation comprising placing low salinity water in the formation.

12. The method of claim 11, further comprising injecting into the resource-containing reservoir (100) zone a fluid having a salinity of from 1400 ppm to 5000 ppm when a percentage of kaolinite is from 0 wt% to 2 wt% and a CTS is present in an amount of from 40 mol% to 90 mol% as determined by high resolution mass spectrometry using atmospheric phase photoionization mode.

13. The method of claim 11, wherein the high resolution mass spectrometry comprises an atmospheric phase photoionization mode.

14. The method of claim 11, wherein the CTS has a double bond equivalent of from 2 to 10.

**Patentansprüche**

1. Verfahren zum Einschätzen der Reaktion eines Lagerstättengesteins auf Wasser mit niedrigem Salzgehalt mit den Schritten:

Gewinnen einer Formationskernprobe (10) eines Lagerstättengesteins aus einer Lagerstätte (100);

sequentielles Waschen der Formationskernprobe (10) mit einer ersten Reihe von Lösungsmitteln, umfassend ein nichtpolares Lösungsmittel, gefolgt von einem polaren Lösungsmittel, um eine erste Reihe von Lösungsmittelextrakten und eine extrahierte Formationskernprobe (10) zu bilden;

sequentielles Waschen der extrahierten Formationskernprobe (10) mit einer Behandlungslösung, um eine zweite Reihe von Lösungsmittelextrakten und eine gereinigte Formationskernprobe (10) zu bilden;

Erzeugen einer Reihe von Massenspektren der zweiten Reihe von Lösungsmittelextrakten, wobei die relative Häufigkeit der Strukturen vom Katecholamintyp (CTS) unter Verwendung der Reihe von Massenspektren bestimmt wird, wobei die CTS organische Moleküle umfassen, die einen aromatischen Kern, bestehend aus einem oder mehreren aromatischen Ringen, und vier oder mehr funktionelle Gruppen an dem aromatischen Kern umfassen, und wobei die CTS aus der Gruppe ausgewählt sind, die aus Verbindungen mit der allgemeinen Formel $C_xH_yN_mO_{x-3}$, wobei x von 10 bis 30 reicht, y von 22 bis 62 reicht und m von 1 bis 2 reicht, und Verbindungen mit der allgemeinen Formel $C_xH_yO_{x-3}$, wobei x von 10 bis 30 reicht und y von 22 bis 62 reicht, besteht;

Durchführen einer Röntgenbeugungsanalyse an der Formationskernprobe (10), um ein Beugungsmuster zu erzeugen, wobei die relative Häufigkeit von Kaolinit unter Verwendung des Beugungsmusters bestimmt wird; und

Bewerten einer Reaktion des Lagerstättengesteins auf Wasser mit niedrigem Salzgehalt basierend auf dem Prozentsatz an Kaolinit und der relativen Häufigkeit von CTS, wobei das Lagerstättengestein mit einem CTS von mehr als 25 Mol-% eine zusätzliche Menge von 0,01 % bis 20 % des ursprünglich vorhandenen Öls liefert, wenn es mit Wasser mit niedrigem Salzgehalt in Kontakt kommt.

2. Verfahren nach Anspruch 1, bei dem das CTS ein Doppelbindungsäquivalent von 2 bis 10 aufweist.

3. Verfahren nach Anspruch 1, bei dem das Lagerstättengestein Quarz, Kaolinit, Illit, Glimmer, Smektit, Chlorit, Feldspat, Plagioklas, Calcit oder eine Kombination davon umfasst.

4. Verfahren nach Anspruch 1, bei dem die Behandlungslösung eine Verbindung mit einem Polaritätsindex im Bereich von 0,175 bis 0,300 umfasst.

**5.** Verfahren nach Anspruch 1, bei dem die Behandlungslösung ein aprotisches Lösungsmittel oder einen aliphatischen cyclischen Ether umfasst.

**6.** Verfahren nach Anspruch 5, bei dem der aliphatische cyclische Ether Dimethylether, Diethylether, einen Dipropylether, einen Dibutylether, Methylethylether, einen Methylpropylether, einen Methylbutylether, Tetrahydrofuran (40), ein substituiertes Tetrahydrofuran, ein Dihydrofuran, ein substituiertes Dihydrofuran, 1,3-Dioxolan, ein substituiertes 1,3-Dioxolan, Tetrahydropyran, ein substituiertes Tetrahydropyran, ein Dihydropyran, ein substituiertes Dihydropyran, Pyran, ein substituiertes Pyran, ein Dioxan, ein substituiertes Dioxan oder eine Kombination davon umfasst.

**7.** Verfahren nach Anspruch 1, bei dem die Behandlungslösung (i) Tetrahydrofuran (40) und/oder (ii) eine Säure oder eine Base umfasst.

**8.** Verfahren nach Anspruch 7, bei dem die Säure Ameisensäure, Essigsäure, Propansäure, Butansäure, Zitronensäure oder eine Kombination davon umfasst oder

bei dem die Säure Ameisensäure umfasst.

**9.** Verfahren nach Anspruch 7, bei dem die Base Kaliumhydroxid, Natriumhydroxid, Pyridin, Alkanamine, Imidazol, Benzimidazol, Histidin, Guanidin, Phosphazen, Hydroxide von quaternären Ammoniumkationen oder eine Kombination davon umfasst, oder bei dem die Base Tetramethylammoniumhydroxid umfasst.

**10.** Verfahren nach Anspruch 1, bei dem die hochauflösende Massenspektrometrie einen Atmosphärenphasen-Photoionisationsmodus umfasst.

**11.** Verfahren zur verbesserten Ölgewinnung aus einer rohstoffhaltigen Zone einer Lagerstätte (100), mit den Schritten:

Gewinnen einer Gesteinsprobe aus der rohstoffhaltigen Zone der Lagerstätte (100);
sequentielles Inkontaktbringen der Gesteinsprobe mit Lösungsmitteln, um Lösungsmittelextrakte und eine gereinigte Gesteinsprobe zu bilden;
Durchführen einer Röntgenbeugung an der gereinigten Gesteinsprobe, wobei die Röntgenbeugung einen Prozentsatz an Kaolinit in der gereinigten Gesteinsprobe identifiziert;
Durchführen einer Massenspektrometrie an den Lösungsmittelextrakten, wobei die Massenspektrometrie einen Prozentsatz an Strukturen vom Katecholamintyp (CTS) in mindestens einem der Lösungsmittelextrakte identifiziert, wobei die CTS organische Moleküle umfassen, die einen aromatischen Kern, bestehend aus einem oder mehreren aromatischen Ringen, und vier oder mehr funktionelle Gruppen in dem aromatischen Kern umfassen, und wobei die CTS Verbindungen mit der allgemeinen Formel $C_xH_yN_mO_{x-3}$, wobei x von 10 bis 30 reicht, y von 22 bis 62 reicht und m von 1 bis 2 reicht, und Verbindungen mit der allgemeinen Formel $C_xH_yO_{x-3}$, wobei x von 10 bis 30 reicht und y von 22 bis 62 reicht, umfassen;
Ermitteln der Lagerstättengesteine mit hoher Wahrscheinlichkeit einer positiven Reaktion auf ein Verfahren zur verbesserten Ölgewinnung mittels Wasser mit niedrigem Salzgehalt basierend auf dem Prozentsatz von Kaolinit und dem Prozentsatz von CTS, wobei eine hohe Wahrscheinlichkeit einer positiven Reaktion durch einen CTS von mehr als 25 Mol-% gekennzeichnet ist; und
Einleiten eines Verfahrens zur verbesserten Ölgewinnung, umfassend das Einbringen von Wasser mit niedrigem Salzgehalt in die Formation.

**12.** Verfahren nach Anspruch 11, ferner umfassend das Einspritzen eines Fluids mit einem Salzgehalt von 1400 ppm bis 5000 ppm in die rohstoffhaltige Lagerstättenzone (100), wenn ein Prozentsatz an Kaolinit von 0 Gew.-% bis 2 Gew.-% vorliegt und ein CTS in einer Menge von 40 Mol-% bis 90 Mol-% vorhanden ist, ermittelt durch hochauflösende Massenspektrometrie unter Verwendung des atmosphärischen Phasen-Photoionisationsmodus.

**13.** Verfahren nach Anspruch 11, wobei die hochauflösende Massenspektrometrie einen atmosphärischen Phasen-Photoionisationsmodus umfasst.

**14.** Verfahren nach Anspruch 11, wobei die CTS ein Doppelbindungsäquivalent von 2 bis 10 aufweist.

**Revendications**

**1.** Procédé d'évaluation de la réponse d'une roche réservoir à de l'eau à faible salinité comprenant :

l'obtention d'un échantillon carotté de formation (10) d'une roche réservoir provenant d'un réservoir (100) ;

le lavage séquentiel de l'échantillon carotté de formation (10) avec une première série de solvants comprenant un solvant non polaire suivi d'un solvant polaire pour former une première série d'extraits de solvant et un échantillon carotté de formation (10) extrait ;

le lavage séquentiel de l'échantillon carotté de formation (10) extrait avec une solution de traitement pour former une deuxième série d'extraits de solvant et un échantillon carotté de formation (10) nettoyé ;

la génération d'une série de spectres de masse de la deuxième série d'extraits de solvant, dans lequel l'abondance relative des structures de type catécholamine (CTS) est déterminée à l'aide de la série de spectres de masse, dans lequel les CTS comprennent des molécules organiques comprenant un noyau aromatique constitué d'un ou plusieurs cycles aromatiques et de quatre groupes fonctionnels ou plus sur le noyau aromatique, et dans lequel les CTS sont sélectionnées dans le groupe consistant en des composés ayant une formule générale $C_xH_yN_mO_{x-3}$ où x varie de 10 à 30, y varie de 22 à 62 et m varie de 1 à 2 et des composés ayant une formule générale $C_xH_yO_{x-3}$ où x varie de 10 à 30 et y varie de 22 à 62 ;

la soumission de l'échantillon carotté de formation (10) à une analyse par diffraction des rayons X pour générer un diagramme de diffraction, dans lequel l'abondance relative de kaolinite est déterminée à l'aide du diagramme de diffraction ; et

l'évaluation d'une réponse de la roche réservoir à de l'eau à faible salinité sur la base du pourcentage de kaolinite et de l'abondance relative de CTS, dans lequel la roche réservoir présentant une CTS supérieure à 25 % en moles produit une quantité supplémentaire de 0,01 % à 20 % du pétrole d'origine en place lorsqu'elle est mise en contact avec de l'eau à faible salinité.

2. Procédé selon la revendication 1, dans lequel la CTS présente un équivalent liaison double de 2 à 10.

3. Procédé selon la revendication 1, dans lequel la roche de réservoir comprend du quartz, de la kaolinite, de l'illite, du mica, de la smécite, de la chlorite, du feldspath, du plagioclase, de la calcite ou une combinaison de ceux-ci.

4. Procédé selon la revendication 1, dans lequel la solution de traitement comprend un composé ayant un indice de polarité allant de 0,175 à 0,300.

5. Procédé selon la revendication 1, dans lequel la solution de traitement comprend un solvant aprotique ou un éther cyclique aliphatique.

6. Procédé selon la revendication 5, dans lequel l'éther cyclique aliphatique comprend un éther diméthylique, un éther diéthylique, un éther dipropylique, un éther dibutylique, un éther de méthyle et d'éthyle, un éther de méthyle et de propyle, un éther de méthyle et de butyle, un tétrahydrofurane (40), un tétrahydrofurane substitué, un dihydrofurane, un dihydrofurane substitué, un 1,3-dioxolane, un 1,3-dioxolane substitué, un tétrahydropyrane, un tétrahydropyrane substitué, un dihydropyrane, un dihydropyrane substitué, un pyrane, un pyrane substitué, un dioxane, un dioxane substitué ou une combinaison de ceux-ci.

7. Procédé selon la revendication 1, dans lequel la solution de traitement comprend (i) un tétrahydrofurane (40) et/ou (ii) un acide ou une base.

8. Procédé selon la revendication 7, dans lequel l'acide comprend de l'acide formique, de l'acide acétique, de l'acide propanique, de l'acide butanoïque, de l'acide citrique ou une combinaison de ceux-ci, ou dans lequel l'acide comprend de l'acide formique.

9. Procédé selon la revendication 7, dans lequel la base comprend de l'hydroxyde de potassium, de l'hydroxyde de sodium, de la pyridine, des alcanamines, de l'imidazole, du benzimidazole, de l'histidine, de la guanidine, du phosphazène, des hydroxydes de cations d'ammonium quaternaire ou une combinaison de ceux-ci, ou dans lequel la base comprend de l'hydroxyde de tétraméthylammonium.

10. Procédé selon la revendication 1, dans lequel la spectrométrie de masse haute résolution comprend un mode de photo-ionisation en phase atmosphérique.

11. Procédé de récupération assistée du pétrole dans une zone de réservoir (100) contenant des ressources comprenant :

l'obtention d'un échantillon de roche provenant de la zone de réservoir (100) contenant des ressources ;

la mise en contact séquentielle de l'échantillon de roche avec des solvants pour former des extraits de solvant et un échantillon de roche nettoyé ;

la soumission de l'échantillon de roche nettoyé à une diffraction des rayons X dans lequel la diffraction des rayons X identifie un pourcentage de kaolinite de l'échantillon de roche nettoyé ;

la soumission des extraits de solvant à une spectrométrie de masse dans lequel la spectrométrie de masse identifie un pourcentage de structures de type catécholamine (CTS) dans au moins l'un des extraits de solvant, dans laquelle les CTS comprennent des molécules organiques comprenant un noyau aromatique constitué d'un ou plusieurs cycles aromatiques et de quatre ou plusieurs groupes fonctionnels dans le noyau aromatique, et dans lequel les CTS comprennent des composés ayant une formule générale $C_xH_yN_mO_{x-3}$ où x varie de 10 à 30, y varie de 22 à 62 et m varie de 1 à 2 et des composés ayant une formule générale $C_xH_yO_{x-3}$ où x varie de 10 à 30 et y varie de 22 à 62 ;

la détermination des roches réservoirs ayant une forte possibilité de comporter une réponse positive à un procédé de récupération assistée du pétrole par eau à faible salinité sur la base du pourcentage de kaolinite et du pourcentage de CTS, dans lequel une forte possibilité de réponse positive est **caractérisée par** le fait de présenter une CTS supérieure à 25 % en moles ; et

le lancement d'une opération de récupération assistée du pétrole comprenant la mise en place d'eau à faible salinité dans la formation.

12. Procédé selon la revendication 11, comprenant en outre l'injection dans la zone de réservoir (100) contenant des ressources d'un fluide ayant une salinité de 1 400 ppm à 5 000 ppm lorsqu'un pourcentage de kaolinite est de 0 % en poids à 2 % en poids et qu'une CTS est présente en une quantité de 40 % en moles à 90 % en moles telle que déterminée par une spectrométrie de masse haute résolution à l'aide du mode de photo-ionisation en phase atmosphérique.

13. Procédé selon la revendication 11, dans lequel la spectrométrie de masse haute résolution comprend un mode de photo-ionisation en phase atmosphérique.

14. Procédé selon la revendication 11, dans lequel la CTS présente un équivalent double liaison de 2 à 10.

FIG. 1

FIG. 2A

FIG. 2B

ROCK B_THF-TMAH

ROCK B

FIG. 2C

FIG. 2D

FIG. 2E

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5741707 A

- US 3208528 A

**Non-patent literature cited in the description**

- **AKSULU HAKAN et al.** *Energy & Fuels*, 2012, vol. 26 (6), 3497-3503
- **CHAVAN MUKUL et al.** *Petroleum Science*, 2019, vol. 16 (6), 1344-1360
- **GUO J. et al.** *Integrated Ocean Drilling Program*, 2011

- **WEBB, K.** ; **LAGER, A.** ; **BLACK C.** Comparison of High/Low Salinity Water/Oil Relative Permeability. *International Symposium of the Society of Core Analysts, Abu Dhabi, UAE*, 29 October 2008